# EUROPEAN PATENT APPLICATION

(11) **EP 1 710 574 A1**
(43) Date of publication of application: **11.10.2006**
(21) Application number: 04807081.7
(22) Date of filing: 15.12.2004
(51) Int. Cl.: G01N 33/15, G01N 33/483, A61P 29/00

(54) **METHOD OF SCREENING COMPOUND SAFE TO GASTRIC MUCOSA**

(30) Priority: 28.01.2004 JP 2004019439
(71) Applicant: LTT Bio-Pharma Co., Ltd., Tokyo 105-6201 (JP)
(72) Inventor: MIZUSHIMA, Toru, 8620949 (JP); MIZUSHIMA, Yutaka, 1060032 (JP)
(74) Representative: Albrecht, Thomas
(86) International application number: PCT/JP2004/018722
(87) International publication number: WO 2005/073718

(57) **Abstract**

A method for screening for compounds or salts thereof, in particular nonsteroidal anti-inflammatory compounds or salts thereof, that are safe for gastric mucosa and cause little gastrointestinal side effects. The method uses a particular liposome to serve as a cell membrane model. The liposome encapsulates a fluorescent dye, in particular, calcein and is formed of phospholipids, such as phosphatidylcholine, phosphatidylglycerol, phosphatidylserine, and phosphatidylinositol. A test compound is allowed to react with the liposome and the leakage of the fluorescent dye from the liposome is evaluated. As a result, compounds safe for gastric mucosa, in particular, anti-inflammatory compounds can be screened.

## Description

### TECHNICAL FIELD

The present invention relates to a method for screening for compounds or salts thereof that are safe for gastric mucosa and causes little gastrointestinal side effects. More particularly, the present invention relates to a method for screening for anti-inflammatory compounds or salts thereof, as well as to a screening method for evaluating/selecting compounds that can protect gastric mucosa.

### BACKGROUND ART

Of different anti-inflammatory drugs, nonsteroidal anti-inflammatory drugs (NSAIDs) are known to be particularly effective in reducing inflammation. However, the ability of these drugs to cause gastrointestinal damage and other serious side effects, in particular, stomach ulcers, poses a problem to the clinical use of these drugs. Specifically, the use of nonsteroidal anti-inflammatory drugs (NSAIDs) is considered the primary cause of stomach gastritis, the currently most prevalent type of stomach ulcer.

More patients are expected to use NSAIDs with the progress of aging society. In fact, as the population ages, NSAIDs have become more widely used to alleviate the symptoms of lumbago, osteoporosis, rheumatoid arthritis, and other diseases.
Thus, the development of NSAIDs that cause no gastrointestinal side effects is an important task. Such NSAIDS are safe for gastric mucosa and have less risk of inducing stomach ulcers.

The present inventors have previously discovered that the ability of NSAIDs to induce stomach ulcers results from the direct cytotoxicity of NSAIDs and have proposed that NSAIDs lacking direct cytotoxicity can serve as useful drugs that are safe for gastric mucosa (Patent Document 1).
[Patent Document 1] Japanese Patent Laid-Open Publication No. 2003-207507

NSAIDs act by inhibiting cyclooxygenase activity in the cell membrane, thereby decreasing prostaglandins that protect gastric mucosa. Also, there have been reports suggesting that some NSAIDs exhibit direct cytotoxicity by inducing necrosis and/or apoptosis. In consideration of these observations, we hypothesized that NSAIDs that inhibit cyclooxygenase activity and do not exhibit direct cytotoxicity can serve as anti-inflammatory drugs that do not cause side effects including stomach ulcers. Based on this hypothesis, we have proposed a screening method that is based on the cytotoxicity to gastric mucosal cells and the inhibition of cyclooxygenase in gastric mucosa.

Our further study based on the hypothesis has led to a new discovery that the membrane toxicity of NSAIDs is responsible for their direct cytotoxicity. We have also succeeded in establishing a cell membrane model that allows easy detection of membrane toxicity of a given NSAID. Thus, this cell membrane model can be used to determine if a given NSAID has membrane toxicity and to search for NSAIDs that are safe for gastric mucosa.
Our cell membrane model can also be used to search for drugs other than NSAIDs that are safe for gastric mucosa, namely, for mucosa-protecting compounds.

### DISCLOSURE OF THE INVENTION

### PROBLEMS TO BE SOLVED BY THE INVENTION

Accordingly, it is an object of the present invention to provide a method for screening for compounds or salts thereof --in particular, nonsteroidal anti-inflammatory compounds or salts thereof-- that are safe for gastric mucosa and cause little gastrointestinal side effects.

### MEANS TO SOLVE THE INVENTION

To achieve the foregoing object, one essential aspect of the present invention comprises the following:
(1) A method for screening for compounds safe for gastric mucosa, comprising preparing liposome serving as a cell membrane model that is formed of a phospholipid and encapsulates a fluorescent dye; allowing a test compound to react with the liposome; and evaluating the leakage of the fluorescent dye from the liposome.
(2) The method for screening according to (1) above, wherein the phospholipid for use in the cell membrane model is selected from the group consisting of phosphatidylcholine, phosphatidylglycerol, phosphatidylserine, phosphatidylinositol, phosphatidylethanolamine, and cardiolipin.
(3) The method for screening according to (1) or (2) above, wherein evaluation of the leakage of the fluorescent dye comprises measuring fluorescence emitted from the dye at an excited wavelength.
(4) The method for screening according to any of (1) to (3) above, wherein the fluorescent dye is selected from the group consisting of calcein, rhodamine, and fluorescein derivatives.
(5) The method for screening according to any of (1) to (3) above, wherein the fluorescent dye is calcein.
(6) The method for screening according to (5), wherein the calcein leakage is determined by measuring fluorescence at 520 nm.

Another aspect of the present invention comprises the following:
(7) The method for screening according to any of (1) to (6) above, wherein the test compound is an anti-inflammatory compound.
(8) The method for screening according to (7) above, wherein the anti-inflammatory compound is a nonsteroidal anti-inflammatory compound or a steroid compound.
(9) The method for screening according to any of (1) to (6) above, wherein the test compound is a compound that acts to protect gastric mucosa.

Still another aspect of the present invention comprises the following:
(10) An anti-inflammatory compound safe for gastric mucosa, obtained by the method for screening according to (7) or (8) above, or a salt thereof.
(11) A gastric mucosa-protecting material, obtained by the method for screening according to (9).

Another aspect of the present invention provides a liposome that serves as a cell membrane model for use in the screening method of the present invention. Specifically, this aspect comprises the following:
(12) A liposome serving as a cell membrane model for use in the screening of compounds having membrane toxicity to gastric mucosa, the liposome being formed of a phospholipid and encapsulating a fluorescent dye.
(13) The liposome according to (12) above, wherein the fluorescent dye is selected from the group consisting of calcein, rhodamine, and fluorescein derivatives.
(14) The liposome according to (12) above, wherein the phospholipid for use in the cell membrane model is selected from the group consisting of phosphatidylcholine, phosphatidylglycerol, phosphatidylserine, phosphatidylinositol, phosphatidylethanolamine, and cardiolipin.

### EFFECT OF THE INVENTION

The screening method of the present invention relies on a simple cell membrane model to detect membrane toxicity of a given drug. Thus, the screening method of the present invention can be used to easily determine if a test compound, in particular a nonsteroidal anti-inflammatory drug (NSAID), has membrane toxicity. As a result, NSAIDs can be obtained that are safe for gastric mucosa and cause no gastrointestinal side effects. Such NSAIDs are of significant clinical importance.

### BRIEF DESCRIPTION OF THE DRAWINGS

Fig. 1 is a diagram showing the correlation between the cytotoxicity (necrosis) of 10 NSAIDs as measured by the decrease in cell viability and their membrane toxicity.
Fig. 2 is a diagram showing the correlation between the cytotoxicity (apoptosis) of 10 NSAIDs as measured by the decrease in cell viability and their membrane toxicity.
Fig. 3 is a diagram showing some of the results of Example 2 of the present invention.
Fig. 4 is a diagram showing some of the results of Example 2 of the present invention.
Fig. 5 is a diagram showing the calcein leakage observed in the presence of indomethacin and teprenone in Example 3 of the present invention.
Fig. 6 is a diagram showing the calcein leakage observed in the presence of diclofenac and teprenone in Example 3 of the present invention.
Fig. 7 is a diagram showing the calcein leakage observed in the presence of celecoxib and teprenone in Example 3 of the present invention.
Fig. 8 is a diagram showing the calcein leakage observed in the presence of ibuprofen and teprenone in Example 3 of the present invention.

### BEST MODE FOR CARRYING OUT THE INVENTION

The cell membrane model for use in the present invention comprises liposome formed of a phospholipid and encapsulating a fluorescent dye that permeates the cell membrane. Since the membrane toxicity of NSAIDs is believed to be largely attributed to the disrupted barrier function of the lipid bilayer of cell membrane, by preparing a liposome formed of a phospholipid, encapsulating a fluorescent dye in the liposome, and allowing NSAIDs to react with the liposome, it is possible to determine the membrane toxicity of a given NSAID by measuring the leakage of the fluorescent dye from the liposome in the presence of the NSAID.

Examples of the fluorescent dye to be encapsulated include calcein, rhodamine, and fluorescein derivatives. Of these, calcein is particularly preferred. Calcein is a fluorescent dye widely used in the determination of cytotoxicity. The dye can stain living cells and has no cytotoxicity itself.

Examples of the phospholipids to form liposome membrane include phosphatidylcholine, phosphatidylglycerol, phosphatidylserine, phosphatidylinositol, phosphatidylethanolamine, and cardiolipin. One or more of these phospholipids may be used to form liposome membrane.

Thus, the present invention uses the means for determining the degree of damage of phospholipid liposome membrane when it is allowed to react with a given test compound, and in other words, uses the phospholipid liposome membrane as an analogue of gastric mucosa.
Therefore, an important issue herein is if there is a certain correlation between the phospholipid liposome membrane and gastric mucosa. The present inventors have demonstrated that the cytotoxicity of NSAIDs due to necrosis/apoptosis as measured by the decrease in cell viability is well-correlated with the membrane toxicity determined by the liposome model.

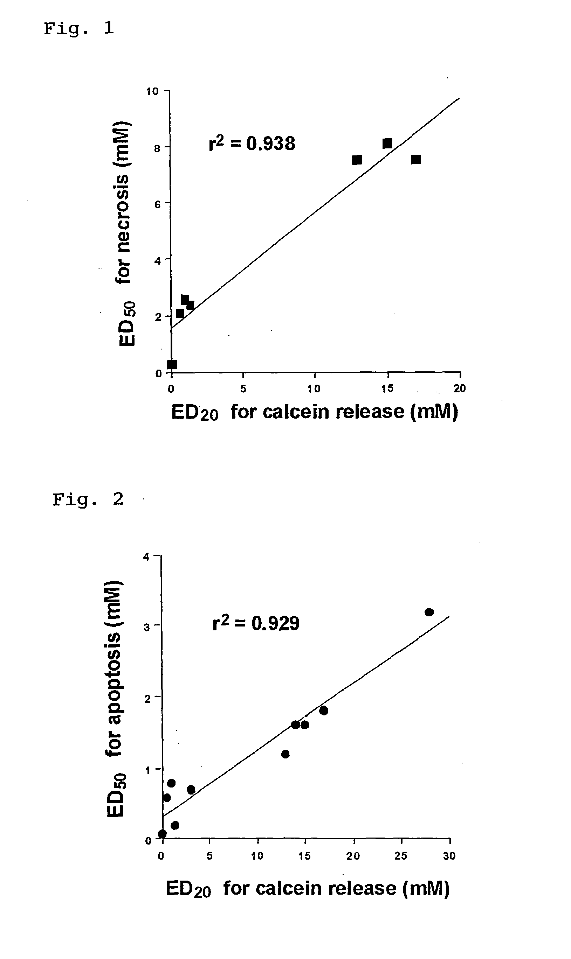

The present inventors have also demonstrated that the membrane toxicity of NSAIDs is responsible for the direct cytotoxicity of NSAIDs. Specifically, the cytotoxicity of 10 clinically used NSAIDs as measured by the decrease in cell viability (i.e., cytotoxicity due to necrosis/apoptosis as measured by the decrease in cell viability) was compared to their membrane toxicity determined by the liposome model. The results shown in Figs. 1 and 2 revealed a high consistency between the cytotoxicity and the membrane toxicity, indicating the direct cytotoxicity of NSAIDs is attributed to their membrane toxicity. In other words, NSAIDs with strong cytotoxicity show strong membrane toxicity and NSAIDs with weak cytotoxicity show weak membrane toxicity (Correlation coefficient = 0.91).

As described above, the cell membrane model of the present invention using liposome allows the determination of membrane toxicity, and thus the cytotoxicity, of NSAIDs. The present inventors have previously discovered that the ability of NSAIDs to induce stomach ulcers is caused by their direct cytotoxicity and have hypothesized that if a given NSAID is proven to lack direct cytotoxicity in the cell membrane model, that NSAID must be safe for gastric mucosa. Since the direct cytotoxicity of NSAIDs results from their membrane toxicity, it is deduced that NSAIDs can be screened for the inability to damage the gastric mucosa by measuring their membrane toxicity in the cell membrane model provided by the present invention.

The screening method of the present invention that screens for NSAIDs that are safe for gastric mucosa by means of the cell membrane model is specifically carried out in the following manner. First, liposome is prepared that is formed of a phospholipid and encapsulates a fluorescent dye such as calcein. NSAIDs to be screened are then allowed to react with the liposome at different concentrations and the leakage of calcein from the liposome (due to the damage to the liposome membrane) is monitored by measuring the fluorescence at 520 nm.

The validity of the screening method for compounds safe for gastric mucosa using the cell membrane model provided by the present invention is demonstrated by that fact that when a mucosa-protecting agent is added to the mixture of the dye-encapsulating, phospholipid liposome and a membrane-toxic NSAID, the leakage of the fluorescent dye from the liposome is significantly reduced.

Specifically, as described clearly in the following examples, when the calcein-encapsulating, phospholipid liposome of the present invention is mixed with one of known membrane-toxic NSAIDs, such as indomethacin, diclofenac, celecoxib and ibuprofen, and teprenone, an antiulcer agent that protects gastric mucosa (product name, Selbex (registered trademark)), the calcein leakage from the liposome is reduced as the amount of teprenone is increased.

This observation suggests that gastric mucosa-protecting teprenone acts to mitigate the membrane toxicity of NSAIDs, demonstrating that compounds, in particular NSAIDs, can be screened for the inability to damage gastric mucosa by measuring their membrane toxicity in the cell membrane model of the present invention.

Although the screening method of the present invention has been described primarily in relation to NSAIDs, it should be appreciated that the method can be used to screen not only for NSAIDs, but also for a wide range of compounds that are safe for gastric mucosa and thus have no membrane toxicity.

### Examples

The present invention will now be described with reference to

### Examples.

### Example 1: Preparation of calcein-encapsulating liposome

Phosphatidylcholine (10 µmol, 7.7 mg) obtained from egg yolk was dissolved in a 1:2 mixture of chloroform/methanol and the solution was dried. The resulting residue was dissolved in 1.5 mL diethylether and the solution was mixed with 1 mL of a 100mM aqueous solution of calcein-sodium hydroxide (pH 7.4). Diethylether was removed to obtain a solution of liposome encapsulating calcein.

### Example 2: Calcein leakage from liposome in the presence of different known NSAIDs

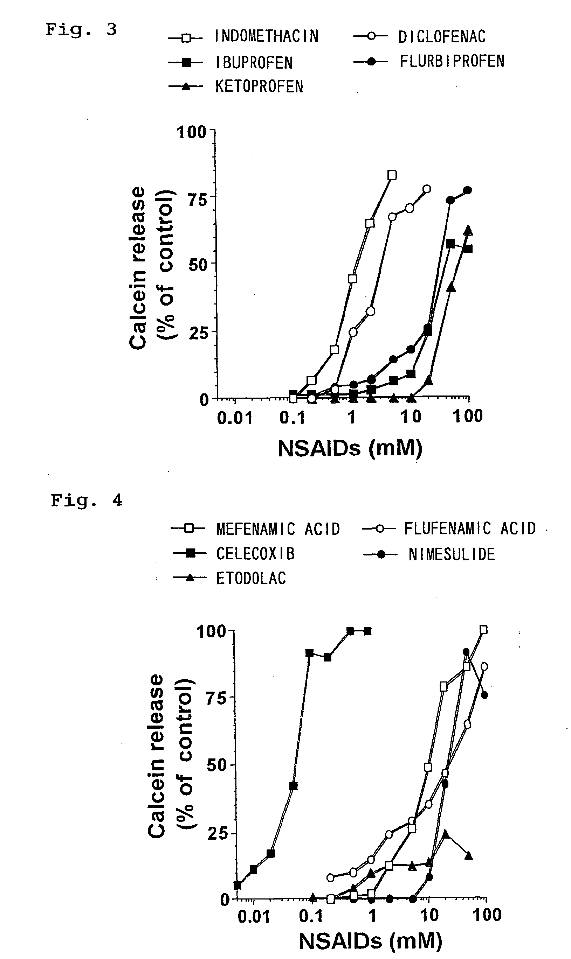

The following clinically used known NSAIDs were used: indomethacin, ibuprofen, ketoprofen, diclofenac, flurbiprofen, mefenamic acid, flufenamic acid, celecoxib, etodolac, and nimesulide. Each NSAID was added at different concentrations to the liposome solution obtained in Example 1 and the mixture was incubated at 30°C for 10 min. Subsequently, the fluorescence at 520 nm was measured to determine the calcein leakage from the liposome as a measure of membrane toxicity.
The results are shown together in Fig. 3 (for indomethacin, ibuprofen, ketoprofen, diclofenac, and flurbiprofen) and Fig. 4 (for mefenamic acid, flufenamic acid, celecoxib, etodolac, and nimesulide).

For indomethacin, ibuprofen, ketoprofen, diclofenac, and flurbiprofen to serve as NSAIDs shown in Fig. 3, the cytotoxicity to gastric mucosal cells decreases in the order of indomethacin > diclofenac > flurbiprofen > ibuprofen > ketoprofen. As can be seen, the calcein leakage from the liposome decreased in the same order. This supports the hypothesis that nonsteroidal anti-inflammatory compounds that are safe for gastric mucosa can be effectively selected by measuring the calcein leakage from the liposome (*i.e.,* membrane toxicity) observed for the compounds.

For mefenamic acid, flufenamic acid, celecoxib, etodolac, and nimesulide to serve as NSAIDs shown in Fig. 4, the cytotoxicity to gastric mucosal cells decreases in the order of celecoxib > mefenamic acid > flufenamic acid > nimesulide > etodolac. As can be seen, the calcein leakage from the liposome decreased in the same order. Similar to the results shown in Fig. 3, this consistency supports the hypothesis that nonsteroidal anti-inflammatory compounds that are safe for gastric mucosa can be effectively selected by measuring the calcein leakage from the liposome (*i.e.,* membrane toxicity) observed for the compounds.

### Example 3: Calcein leakage from liposome in the presence of different known NSAIDs and gastric mucosa-protecting agent

The following clinically used known NSAIDs were used: indomethacin, diclofenac, celecoxib, and ibuprofen. The calcein leakage from the liposome was examined in the presence of one of the NSAIDs and an agent that acts to protect gastric mucosa.

Method:
Each of the NSAIDs was added at a concentration high enough to exhibit significant cell membrane toxicity: 6 mM for indomethacin, 4 mM for diclofenac, 0.08 mM for celecoxib and 20 mM for ibuprofen.
Teprenone (product name, Selbex (registered trademark)) was used as the gastric mucosa-protecting agent and was used at concentrations of 0 M (Control), 10⁻⁷ M, 10⁻⁶ M, 10⁻⁵ M and 10⁻⁴ M.
Each NSAID and teprenone at respective concentrations were added to the liposome solution obtained in Example 1. Each solution was incubated at 30°C for 10 min. Subsequently, the fluorescence at 520 nm was measured for each solution to determine the calcein leakage from liposome (i.e., membrane toxicity).

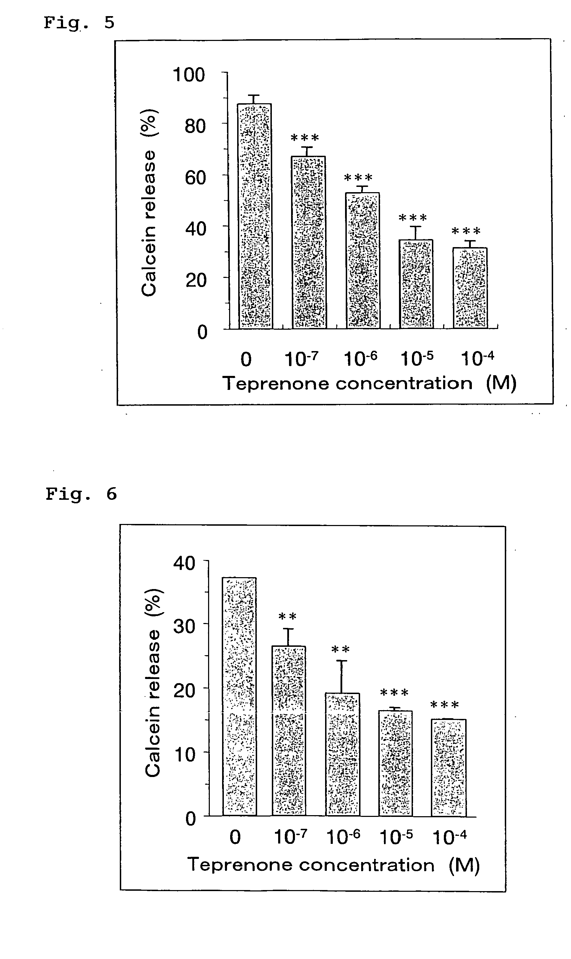

Results:
The results are shown in Figs. 5 through 8.
Fig. 5 shows the calcein leakage in the presence of 6 mM indomethacin and varying concentrations of teprenone. It is seen that the calcein leakage decreases as the amount of teprenone is increased.

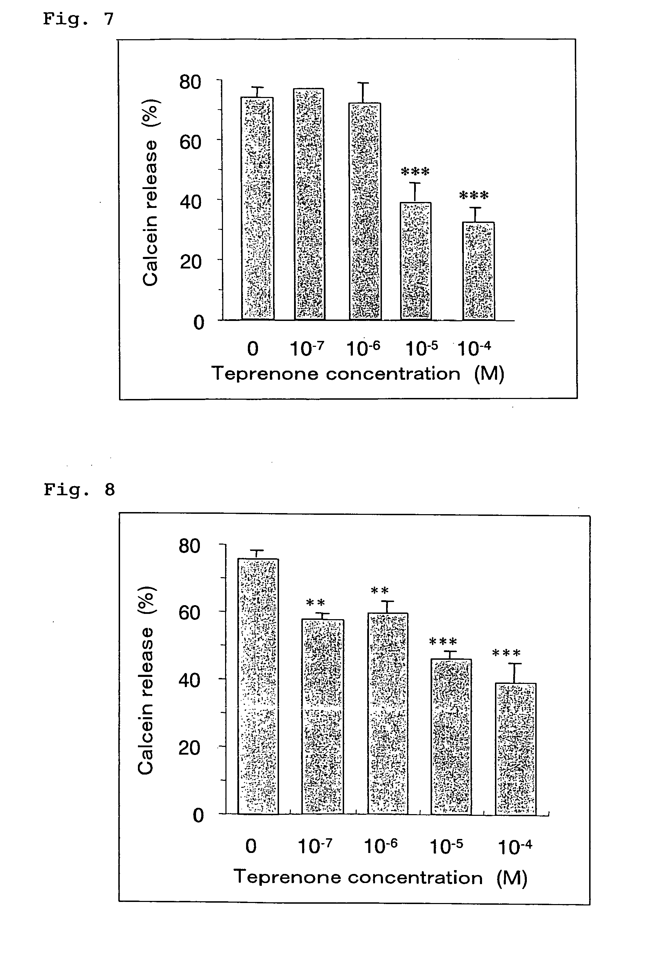

Fig. 6 shows the calcein leakage in the presence of 4 mM diclofenac and varying concentrations of teprenone. Fig. 7 shows the calcein leakage in the presence of 0.08 mM celecoxib and varying concentrations of teprenone. Fig. 8 shows the calcein leakage in the presence of 20 mM ibuprofen and varying concentrations of teprenone. As can be seen from each figure, the calcein leakage decreases as the amount of teprenone is increased.

The same experiment was repeated by varying the concentration of each of the 4 NSAIDs: 4 mM and 8 mM for indomethacin, 2 mM and 10 mM for diclofenac, 0.05 mM and 0.1 mM for celecoxib, and 40 mM and 60 mM for ibuprofen. In each case, the co-presence of teprenone reduced the calcein leakage from the liposome (i.e., membrane toxicity).

### INDUSTRIAL APPLICABILITY

As set forth, the simple cell membrane model used in the screening method of the present invention enables detection of the membrane toxicity of test compounds, in particular nonsteroidal anti-inflammatory drugs (NSAIDs). The screening method of the present invention has a significant advantage in that it allows the development of NSAIDs and other clinically useful compounds that exhibit little membrane toxicity and are safe for gastric mucosa.

## Claims

1. A method for screening for compounds safe for gastric mucosa, comprising:
preparing liposome serving as a cell membrane model that is formed of a phospholipid and encapsulates a fluorescent dye;
allowing a test compound to react with the liposome; and
evaluating the leakage of the fluorescent dye from the liposome.

2. The method for screening according to claim 1, wherein the phospholipid for use in the cell membrane model is selected from the group consisting of phosphatidylcholine, phosphatidylglycerol, phosphatidylserine, phosphatidylinositol, phosphatidylethanolamine, and cardiolipin.

3. The method for screening according to claim 1 or 2, wherein evaluation of the leakage of the fluorescent dye comprises measuring fluorescence emitted from the dye at an excited wavelength.

4. The method for screening according to any of claims 1 to 3, wherein the fluorescent dye is selected from the group consisting of calcein, rhodamine, and fluorescein derivatives.

5. The method for screening according to any of claim 1 to 3, wherein the fluorescent dye is calcein.

6. The method for screening according to claim 5, wherein the calcein leakage is determined by measuring fluorescence at 520 nm.

7. The method for screening according to any of claims 1 to 6, wherein the test compound is an anti-inflammatory compound.

8. The method for screening according to claim 7, wherein the anti-inflammatory compound is a nonsteroidal anti-inflammatory compound or a steroid compound.

9. The method for screening according to any of claims 1 to 6, wherein the test compound is a compound that acts to protect gastric mucosa.

10. An anti-inflammatory compound safe for gastric mucosa, obtained by the method for screening according to claim 7 or 8, or a salt thereof.

11. A gastric mucosa-protecting material, obtained by the method for screening according to claim 9.

12. A liposome serving as a cell membrane model for use in the screening of compounds having membrane toxicity to gastric mucosa, the liposome being formed of a phospholipid and encapsulating a fluorescent dye.

13. The liposome according to claim 12, wherein the fluorescent dye is selected from the group consisting of calcein, rhodamine, and fluorescein derivatives.

14. The liposome according to claim 12, wherein the phospholipid for use in the cell membrane model is selected from the group consisting of phosphatidylcholine, phosphatidylglycerol, phosphatidylserine, phosphatidylinositol, phosphatidylethanolamine, and cardiolipin.
